# FASCICULE DE BREVET EUROPEEN

(11) **EP 3 277 295 B1**
(45) Date de publication et mention de la délivrance du brevet: **28.04.2021**
(21) Numéro de dépôt: 16717275.8
(22) Date de dépôt: 31.03.2016
(51) Int. Cl.: A23L 33/18, A23L 33/19, A61K 31/185, A61K 35/20, A61P 1/00, A61P 3/00, A61P 19/02, A61P 25/28, A61P 37/00, A61P 35/00, A61K 31/198, A61K 31/201, A61K 31/202, A61K 31/355, A61K 31/405, A61K 31/4415, A61K 31/59, A61K 31/519, A61K 33/06, A61K 33/24

(54) **COMPOSITION POUR LE TRAITEMENT D'UN ETAT METABOLIQUE PATHOGENE DU MICROBIOTE INTESTINAL ET DES MALADIES DERIVEES**
ZUSAMMENSETZUNG ZUR BEHANDLUNG EINES PATHOGENEN STOFFWECHSELZUSTANDES DER DARMFLORA UND DARAUS FOLGENDEN ERKRANKUNGEN
COMPOSITION FOR THE TREATMENT OF A PATHOGENIC METABOLIC STATE OF THE INTESTINAL MICROBIOTA AND OF DERIVED DISEASES

(30) Priorité: 31.03.2015 FR 1552740; 30.04.2015 US 201514700462
(43) Date de publication de la demande: 07.02.2018
(73) Titulaire: Ysopia Biosciences, 33000 Bordeaux (FR)
(72) Inventeur: VINCENT, Claude, 33000 Bordeaux (FR)
(74) Mandataire: Aquinov
(86) Numéro de dépôt international: PCT/EP2016/057128
(87) Numéro de publication internationale: WO 2016/156527

(56) Documents cités:
- FR-A1- 2 981 544
- FR-A1- 2 981 545
- FR-A1- 3 007 985
- GOODRICH JULIA K ET AL: "Human Genetics Shape the Gut Microbiome", CELL, CELL PRESS, US, vol. 159, no. 4, 6 novembre 2014 (2014-11-06), pages 789-799, XP029095122, ISSN: 0092-8674, DOI: 10.1016/J.CELL.2014.09.053
- L S MORTENSEN ET AL: "Effects of different fractions of whey protein on postprandial lipid and hormone responses in type 2 diabetes", EUROPEAN JOURNAL OF CLINICAL NUTRITION, vol. 66, no. 7, 16 mai 2012 (2012-05-16), pages 799-805, XP055217581, ISSN: 0954-3007, DOI: 10.1038/ejcn.2012.48

## Description

La présente invention concerne l'utilisation d'une composition comme médicament ou produit de nutrition médicale pour le traitement d'un état métabolique pathogène du microbiote intestinal provoqué par une dysbiose pathologique d'origine métabolique et/ou pour la prévention et le traitement de maladies dérivées de cet état pathogène.

Le microbiote est un ensemble de microorganismes (bactéries, archaéas, virus et eucaryotes) qui sont spécifiques à chacun des individus. Ces microorganismes se trouvent sur la peau, dans la bouche et le plus grand nombre dans le système digestif qui compte pour 10¹² cellules par gramme et comme il existe 1.5 à 2 kg de microbiote, la présence d'espèces différentes se comptent par millions soit de 3.3 à 8 millions selon les experts, et la totalité des microorganismes en milliards. Ce microbiote contient plus de cent cinquante fois de gènes que le génome de l'hôte.

Les microorganismes du microbiote intestinal sont classés par domaine (par exemple bactéries), phylum (par exemple *Firmicutes),* classe (par exemple *Clostridia*), ordre (par exemple *Clostridiales*)*,* famille (par exemple *Christensenellaceae*), genre (par exemple *Christensenella*) et espèce (par exemple *Christensenella minuta*)*.*

Chaque individu a son propre microbiote qui provient de son histoire et de ses racines.

Toutefois dans une cohorte, 75% des espèces se retrouvent dans 50% de la cohorte et 57% dans 90% de la cohorte. De plus 85% des espèces sont communes entre l'Europe, les USA et le Japon.

Le microbiote intestinal n'est pas homogène. Pour un même individu il varie en quantité et qualité depuis l'estomac (10¹ avec *lactobacillus, vellonella, helicobacter)* au duodénum, jéjunum, iléum (10³ à 10⁷ avec *bacilli, streptococcaceae, actinobacteria, actinomycinaeae, corynebacteriaeae*) et enfin le colon (10¹² avec *lachnospiraceae, bactériodetes*)*.* Certaines bactéries (*clostridium, lactobacillus, enterococcus*) sont également trouvées dans le mucus qui tapisse la paroi intestinale. Les grands phyla sont relativement stables chez un individu et les différences se situent au niveau des espèces soit souvent quelques pourcents du microbiote total. Ainsi les états pathogènes sont difficiles à déceler car provenant de ces espèces particulières.

Les anomalies de l'hôte (génétique et environnementales) se retrouvent dans une flore déséquilibrée pathogène appelée dysbiose pathogène ; le seuil de déséquilibre est difficile à fixer, de même que les variations des genres et espèces sauf s'il existe un biomarqueur de l'anomalie ou de la maladie.

Depuis quelques années, on sait que le microbiote intestinal n'est pas uniquement impliqué dans la digestion et la transformation de la nourriture en énergie, mais qu'il joue un rôle majeur dans le maintien de la bonne santé et dans l'apparition de plusieurs maladies. Des récentes recherches laissent penser que le comportement du cerveau, le système entéro-endocrinien ou neurovégétatif ou encore les dégénérescences sont pilotés par le microbiote.

On sait également que le microbiote intestinal est un régulateur majeur de l'immunité et participe intensément à la régulation de l'expression génique de l'hôte.

A travers la dysbiose, c'est-à-dire un changement dans la composition du microbiote, le microbiote peut être associé à des désordres métaboliques pathologiques dérivés tels que le diabète de type 2 ou bien les maladies cardiovasculaires. Par ailleurs, certaines composantes du microbiote ont été associées à d'autres maladies comme les maladies dégénératives, intestinales et certains cancers.

Le microbiote intestinal est également en corrélation directe avec le poids de l'hôte. Le surpoids avec comorbidités et l'obésité, qui se définissent par un indice de masse corporelle (IMC) de 25 à 29,9 pour le surpoids et supérieur à 30 pour l'obésité, sont en effet associés à une dysbiose pathologique d'origine métabolique du microbiote intestinal.

Le surpoids avec des comorbidités, l'obésité et les maladies métaboliques au sens large, créent des ravages dans nos sociétés modernes que ce soit dans les pays à développement avancé, dans les pays émergents ou même les pays en voie de développement.

La recherche des raisons de cette épidémie a pris une importance cruciale afin de ne pas connaître une chute de l'espérance de vie dans les 25 prochaines années et surtout pour éviter un élargissement de la plage de fin de vie en mauvaise santé et dépendance.

Les causes fondamentales de la prise de poids sont un déséquilibre de la prise alimentaire en particulier de lipides saturés, de fructose et d'hydrates de carbone associés ou non à une sédentarité importante par rapport aux prises alimentaires, c'est-à-dire par rapport à la quantité d'énergie. Toutefois, chacun n'est pas égal devant la même prise alimentaire. Il existe en effet des différences en particulier au niveau de l'absorption et du stockage de l'énergie sous forme de gras, et ces différences sont en lien avec l'état pathogène du microbiote intestinal.

En cas de surpoids et d'obésité, on a pu constater que :
- la richesse et diversité du microbiote diminue
- certaines familles, genres et espèces pathogènes prennent le pas sur d'autres
- le microbiote pathogène a la capacité d'extraire plus d'énergie des aliments même de fibres non digestibles.

En outre, chez les personnes en surpoids et obèses, une restriction calorique avec un rééquilibrage alimentaire augmente la richesse totale de la flore par la perte de masse grasse mais il existe une différence de résultats du régime selon la richesse de la flore de départ. Pour les microbiotes pauvres au départ, les résultats sont inférieurs, en particulier sur la diminution de l'inflammation et de l'insulinorésistance.

Par ailleurs, l'analyse du microbiote de personnes minces ou même anorexiques par rapport aux obèses a établi une corrélation avec certains microorganismes.

Une corrélation entre les phyla et le surpoids/obésité a été recherchée, et une relation a été trouvée entre les 2 plus grandes phyla : le rapport relatif *Firmicutes* sur *Bactériodetes* augmenterait avec l'IMC et l'effet sur la perte de poids se traduirait par la diminution de ce rapport (Damms-machado a 2015, Pekkala s 2015, Remely m 2015, eslinger aj 2014, Bervoets I 2013, Fava f 2013), mais ce constat a été aussi infirmé par des résultats démontrant le contraire (Rahat-Rozenbloom, 2014 Xu P2012, Sefcíková Z 2010).

On sait que la composition du microbiote d'un individu est relativement homogène dans le temps au niveau des phyla (sauf incidents passagers comme un traitement antibiotique) mais que les genres peuvent varier, et on sait également qu'entre plusieurs individus, les phyla varient en fonction de l'âge, de la géolocalisation, de l'ethnie, du mode de vie, etc. Il est donc impossible d'établir un microbiote standard de bonne santé, mais il est possible de rechercher quels sont les genres et espèces qui marquent un état sans ou avec pathologie.

Certains entérotypes avec assemblage de relations positives ou négatives ont été déterminés. En particulier trois entérotypes dominent dans le monde selon la primauté du phylum le plus important : bactériodetes, provotella et ruminococcus, mais aucune corrélation directe n'a pu être établie entre ces entérotypes et les maladies métaboliques.

Pour la constitution du microbiote jusqu'en 2012, il était admis que le microbiote se construisait par l'influence externe et qu'il était entièrement acquis de 0 à 3 ans par l'environnement et la diète. Dans une étude de novembre 2014, une équipe internationale dirigée par Ruth Ley (Cornell University NY) a publié sous la direction de Julia Goodrich un article (« Human genetic shape the gut microbiome » Cell november 6, 159 789-799) qui infirme le concept unique de l'acquisition en introduisant pour quelques familles, genres et espèces la notion d'héritabilité ; elle a fait cette constatation en étudiant une cohorte de vrais et faux jumeaux et des personnes témoins. Les souches du microbiote intestinal démontrées comme héritables sont :
- des familles de bactéries dans les *Firmicutes : lachanospiraceae, ruminococcaceae et christensenellaceae,*
- une archéa dans les *Méthanogènes* : M. smithii.

La famille des *Christensenellaceae* est la famille la plus transmissible. Elle comprend en particulier le genre *Christensenella* et notamment l'espèce *Christensenella minuta* qui a été définie et cultivée récemment par Masami Morotomi à Tokyo décrite dans son étude « Description of christensenella minuta gen. nov.sp.nov. isolated from human faeces, which forms a distinct branch in the other clostridiales, and proposal of christensenellaceae » (M Morotomi 2012 inter. Jour. Of systematic and evolution microbiology 62 144-149).

Chez l'enfant, à la naissance, la richesse de *Christensenellaceae* est de 20% dans le méconium contre 8.6% pour la mère. Cette proportion baisse relativement ensuite (pour être environ de 5 %) par l'augmentation rapide des autres phyla en particulier celles qui vont devenir prépondérantes comme *Firmicutes, Bactériodetes, Protéobacteria* et *Actinobacteria* avec l'introduction des aliments (par exemple le gras animal ou les protéines) et l'environnement).

La présence de *Christensenella* est associée à un IMC bas ainsi qu'à un état de « bonne santé». Plus la quantité relative de *Christensenella* est importante, plus l'hôte a tendance à être maigre et en bonne santé. *Christensenella* deviendrait, par la faiblesse de sa présence relative, le biomarqueur de la dysbiose pathogène, état pathologique métabolique conduisant en particulier au surpoids avec comorbidités, à l'obésité et à des maladies métaboliques.

La famille des *christensenellaceae* est le chef de file d'un « consortium » composé des familles suivantes classées par ordre d'importance décroissante dans l'action métabolique : *dehalobacteriaceae* (firmicutes), *clostridiales* non classées, *ténericutes* genres non classés ML615J-28 et RF39, *méthanobacteriaceae* (euryarchaeota), *firmicutes* genre non classé *SHA-98, peptococcaceae, verrucomicrobiaceae.*

La famille des *Christensenellaceae* est souvent liée positivement à l'abondance de *Méthagènes* qui est elle-même corrélée à la production de butyrate et propionate, mais *Christensenella* n'a pas besoin de cette liaison pour s'exprimer dans le métabolisme car elle contribue directement au phénotype de l'hôte à qui elle est associée.

Julia Goodrich (« Human genetic shape the gut microbiome » Cell november 6, 159 789-799) a continué son observation chez les souris germ-free ensemencées par du microbiote d'obèse en 2 groupes dont un seul reçoit une implantation de *Christensenella,* cette présence occasionne une prise de poids inférieure de 30%.

Le genre *Christensenella* est donc reconnu comme marqueur de la maigreur lorsqu'il est présent en quantité suffisante dans le microbiote intestinale. D'autre part, il est le marqueur du surpoids et de l'obésité lorsqu'il est présent en faible quantité dans le microbiote intestinal. L'augmentation relative de *Christensenella* est le garant d'une baisse de poids en particulier sur la masse maigre et le moteur d'une amélioration métabolique.

Par ailleurs, ces bactéries *Christensenella* seraient impliquées dans d'autres maladies métaboliques comme le diabète, les maladies cardiaques et vasculaires, l'athérosclérose, les maladies dégénératives osseuses, les maladies neurodégénératives, les cancers liés au métabolisme, les maladies auto-immunes et les stéatoses et stéatohépatites métaboliques.

Actuellement, il existe un besoin pour un produit capable d'agir sur le microbiote intestinal pour pouvoir être utilisé pour le traitement du microbiote pathogène et par conséquent pour la prévention et le traitement de maladies métaboliques dérivées, tels que le surpoids avec comorbidités, l'obésité, etc.

Pour y répondre, la présente invention propose d'utiliser une composition capable d'augmenter la quantité relative de *Christensenella* dans le microbiote intestinal.

Une telle composition est déjà connue pour des applications différentes. Ainsi, il est décrit dans les brevets FR 2981544 et FR 2981545 son utilisation pour respectivement réduire la graisse viscérale et le gras sous-cutané profond chez l'être humain et réduire la graisse viscérale en préopératoire bariatrique pour des patients atteints d'obésité.

Il est également connu dans la demande de brevet FR3007985 une composition comprenant un hydrolysat de lactosérum, de l'alpha lactalbumine, un principe actif pharmaceutique et un régulateur nutritionnel. Or, une telle composition n'est pas satisfaisante pour le traitement d'un état pathologique du microbiote.

A cet effet, l'invention vise l'utilisation d'une composition comprenant un mélange de principes actifs constitué par au moins :
- un hydrolysat de lactosérum de poids moléculaire compris entre 200 et 10000 daltons,
- un isolat et/ou un concentrat de lactosérum de poids moléculaire compris entre 15 000 et 20 000 Daltons, et
- du caséinate de calcium,
pour son utilisation comme médicament ou produit de nutrition médicale chez l'homme pour le traitement d'un état pathologique du microbiote, à savoir d'une dysbiose pathologique métabolique caractérisée par une baisse de *Christensenella* dans le microbiote intestinal.

La composition peut être utilisée également pour la prévention ou le traitement d'au moins une maladie métabolique dérivée, en particulier d'une maladie métabolique marquée par une déficience en *Christensenella* dans le microbiote intestinal, notamment une maladie métabolique marquée par une déficience en *Christensenella* dans le microbiote intestinal choisie parmi le surpoids avec comorbidités, l'obésité, le diabète, les maladies cardiaques et vasculaires, l'athérosclérose, les maladies neurodégénératives, les cancers liés au métabolisme, les maladies auto-immunes, la stéatose, la stéatohépatite métabolique et les maladies inflammatoires chroniques de l'intestin. La composition traite le microbiote pathologique en particulier en augmentant relativement dans l'intestin le genre bactérien *Christensenella* (phylum *Firmicutes,* classe *Clostridia,* ordre *Clostridiales,* famille *Christensenellaceae,* genre *Christensenella*).

Avantageusement, une telle composition est capable d'augmenter relativement la quantité de *Christensenella* dans le microbiote intestinal afin de combattre une pathogénèse métabolique. Elle peut ainsi être utilisée comme médicament ou produit de nutrition médicale pour rééquilibrer le microbiote intestinal par l'augmentation du genre *Christensenella* et pour prévenir et lutter contre les maladies découlant de ce déséquilibre pathologique du microbiote intestinal.

L'invention est à présent décrite en détails, la description du dispositif étant établie en regard des dessins annexés, dessins sur lesquels les différentes figures montrent :
- Figure 1 : un schéma des résultats du tableau 1 relatifs à l'abondance relative en *Christensenella,*
- Figure 2 : un tableau en codes à barre, réalisé par E. Prifti, des résultats obtenus sur l'étude réalisée sur le microbiote intestinal de patients traités avec un placebo et de patients traités selon l'invention.

L'invention a donc pour objet une composition comprenant un mélange de principes actifs constitué par au moins :
- un hydrolysat de lactosérum de poids moléculaire compris entre 200 et 10000 daltons,
- un isolat et/ou un concentrat de lactosérum de poids moléculaire compris entre 15 000 et 20 000 Daltons, et
- du caséinate de calcium,
pour son utilisation comme médicament ou produit de nutrition médicale chez l'homme pour le traitement d'une dysbiose pathologique caractérisée par une déficience en *Christensenella* dans le microbiote intestinal et/ou pour la prévention ou le traitement d'au moins une maladie métabolique dérivée de cet état pathologique du microbiote intestinal choisie parmi le surpoids avec comorbidités, l'obésité, le diabète, les maladies cardiaques et vasculaires, l'athérosclérose, les maladies dégénératives osseuses, les maladies neurodégénératives, les cancers liés au métabolisme, les maladies auto-immunes, la stéatose, la stéatohépatite métabolique, et les maladies inflammatoires chroniques de l'intestin.

La composition agit par l'augmentation relative dans l'intestin du genre bactérien *Christensenella.*

La composition est connue, mais de façon surprenante et inattendue, elle agit sur le microbiote et en particulier elle est capable d'augmenter *Christensenella.*

Par «déficience » en *Christensenella* dans le microbiote intestinal, au sens de l'invention on entend un niveau de *Christensenella* dans le microbiote intestinal représentant moins de 0.01% du total des genres bactériens décelés dans le microbiote d'un individu donné (recueilli dans les selles, l'abondance étant mesurée de préférence par un test de séquençage Fish, ou de qPCR ou de méta analyse).

Par « *Christensenella »* on entend les bactéries du phylum *Firmicutes,* de la classe *Clostridia,* de l'ordre *Clostridiales,* de la famille *Christensenellaceae,* et du genre *Christensenella.*

Par « composition de nutrition médicale » ou « produit de nutrition médicale » ou « *medical food* » ou denrées alimentaires destinées à des fins médicales spéciales (DADFMS) ou Aliments diététiques destinés à des fins médicales spéciales (ADDFMS), au sens de l'invention, on entend un aliment à vocation thérapeutique de prévention ou de traitement, utilisé seul ou associé à d'autres thérapies. Il s'agit d'un composé nutritionnel, répondant à une situation clinique particulière, susceptible de constituer l'alimentation exclusive ou partielle des patients auxquelles elle est destinée.

Par « concentrat de lactosérum » au sens de l'invention on entend un extrait de lactosérum obtenu par concentration d'un lactosérum.

Par « dysbiose pathologique » au sens de l'invention on entend un état pathologique caractérisé par un déséquilibre dans la répartition des bactéries du microbiote intestinal au niveau des phyla, classes, ordres, familles, genres ou espèces, conduisant à un risque d'autres maladies dont l'origine est d'ordre métabolique.

Par « hydrolysat de lactosérum » au sens de l'invention on entend toute molécule ou mélange de molécules obtenu(e) par un procédé comprenant une étape d'hydrolyse chimique ou d'hydrolyse enzymatique de lactosérum.

Par « isolat de lactosérum » au sens de l'invention on entend un extrait de lactosérum qui contient moins de 1% de lactose et de matières grasses.

Par « médicament » au sens de l'invention on entend un produit ayant obtenu une autorisation de mise sur le marché comme produit de traitement d'une maladie.

Par « microbiote » au sens de l'invention on entend le microbiote intestinal.

L'hydrolysat de lactosérum de la composition selon l'invention a un poids moléculaire compris entre 200 et 10 000 Daltons, préférentiellement entre 200 et 3500 Daltons. Il est essentiellement composé de di et tri peptides.

De façon préférée, il s'agit d'un hydrolysat peptidique de lactosérum comprenant au moins 90% de peptides en poids de matière sèche de l'hydrolysat.

L'isolat et/ou le concentrat de lactosérum ont préférentiellement un poids moléculaire compris entre 15 000 et 20 000 Daltons.

L'isolat de lactosérum est préférentiellement fabriqué à partir de lait frais et de fromageries qui ne pasteurisent pas le lait pour éviter la destruction des bêta-lactoglobuline et alpha-lactalbumine, et qui extraient le lactosérum par ultrafiltration ou microfiltration (taille des filtres de 0,1µm). L'isolat obtenu par échange d'ions est moins adapté en raison de sa faible teneur en bêta-lactoglobuline et alpha-lactalbumine. L'isolat contient moins de 1% de lactose et de matières grasses, et sa concentration en peptides est préférentiellement d'au moins 90% en poids de matière sèche.

Le concentrat de lactosérum est préférentiellement obtenu à partir d'un lactosérum de fromagerie sans pasteurisation, contenant des bêta-lactoglobuline, des alpha-lactalbumine et des glycomacropetides. La concentration en peptides du concentrat est préférentiellement d'au moins 80% en poids de matière sèche.

Par ailleurs, le caséinate de calcium utilisé dans la composition selon l'invention a préférentiellement un poids moléculaire compris entre 20 000 et 35 000 daltons.

De façon préférée, le ratio en poids entre le caséinate de calcium et le mélange constitué par l'hydrolysat et l'isolat et/ou le concentrat de lactosérum est de 0,8 à 1,2 dans la composition.

Selon un mode de réalisation particulièrement adapté, le mélange de principes actifs de la composition comprend également un mélange d'acides aminés.

Les acides aminés présents dans la composition sont préférentiellement au moins le tryptophane, la glutamine, la leucine, l'arginine et/ou la taurine, mais la composition peut contenir d'autres acides aminés, comme l'isoleucine, la valine, la phénylalanine ou la thréonine. Très préférentiellement, la composition selon l'invention comprend au moins le tryptophane, la leucine, l'arginine et la taurine.

Lorsque le tryptophane est présent, il doit représenter entre 6 et 9%, préférentiellement environ 7% en poids des acides aminés neutres présents dans la composition (leucine, isoleucine, valine, phénylalanine, tyrosine et tryptophane).

Lorsque l'arginine et la taurine sont présentes, le rapport du poids d'arginine sur le poids de taurine est compris entre 1,5 et 2.

En plus du mélange hydrolysat, isolat et/ou concentrat de lactosérum et caséinate de calcium, et des acides aminés, le mélange de principes actifs de la composition selon l'invention peut comprendre un ou plusieurs des éléments choisis parmi le calcium de lait, le magnésium, la vitamine B6, la vitamine B9, la vitamine E, la vitamine D, le zinc et le chrome.

De même, la composition peut contenir des acides gras essentiels, notamment des omégas 3. Préférentiellement, il s'agit d'omégas 3 d'origine végétale, avec une proportion élevée d'EPA.

Selon un mode de réalisation préféré, le mélange de principes actifs de la composition selon l'invention comprend au moins :
- 8 à 12 % d'hydrolysat de lactosérum,
- 15 à 20 % d'isolat et/ou de concentrat de lactosérum,
- 20 à 25 % de caséinate de calcium.
les pourcentages étant donnés en poids de matière sèche de la totalité des principes actifs présents de la composition (en dehors des éventuels excipients).

La composition peut également contenir des éléments ajoutés librement, tels que des acides aminés, des vitamines et des minéraux, qui viennent s'ajouter aux constituants natifs de l'hydrolysat de lactosérum, de l'isolat de lactosérum, du concentrat de lactosérum et du caséinate de calcium.

La composition selon l'invention est préférentiellement constituée par au moins :
- 1,5 à 3% de tryptophane,
- 12 à 20 % d'acides aminés branchés,
- 6 à 10 % d'acides aminés aromatiques,
- 0,8 à 1,5% de taurine,
- 1,6 à 3% d'arginine,
- 1,2 à 3% de calcium de lait,
- 0,5 à 1% de magnésium,
- 0,4 à 1% d'omégas 3,
- 1 à 2 mg de vitamine B6 pour 50g de composition sans excipients,
- 5 à 15mg de zinc pour 50g de composition sans excipients,
- 1 à 3µg de vitamine D pour 50g de composition sans excipients,
- 75 à 150 µg de chrome pour 50g de composition sans excipients,
- 100 µg de vitamine B9 pour 50g de composition sans excipients,
- 10mg de vitamine E pour 50g de composition sans excipients.
les pourcentages étant donnés en poids de matière sèche de la totalité des principes actifs présents de la composition (en dehors des éventuels excipients), une partie des constituants provenant de l'hydrolysat de lactosérum, de l'isolat de lactosérum, du concentrat de lactosérum et du caséinate de calcium et le reste étant ajouté librement sous forme d'acides aminés, vitamines et minéraux.

Les acides aminés branchés de la composition sont constitués de leucine, d'isoleucine et de valine, préférentiellement de :
- 50 à 60% de leucine,
- de 18 à 25% d'isoleucine, et
- de 20 à 28% de valine,
et les acides aminés aromatiques de tryptophane, de phénylalanine et de tyrosine, préférentiellement de :
- 15 à 24% de tryptophane,
- de 38 à 46% de phénylalanine, et
- de 35 à 43% de tyrosine.

La composition selon l'invention peut être obtenue par un procédé tel que décrit en suivant :
- un premier mélange est obtenu par mélange des constituants dans l'ordre suivant : caséinate de calcium, isolat de lactosérum, concentrat de lactosérum, hydrolysat de lactosérum, acides aminés libres, magnésium et calcium de lait. Le pH doit être aux alentours de 7 et stabilisé à ce niveau.
- ajout au premier mélange des vitamines, des minéraux et des acides gras.

On obtient ainsi une poudre qui peut être transformée en comprimé ou liquide, ou bien utilisée dans sa forme poudre en sachets, sticks, bidons ou gélules par exemple.

La composition selon l'invention, lorsqu'elle est administrée par voie orale en quantité suffisante, permet de modifier la composition du microbiote intestinal en augmentant relativement la quantité de *Christensenella* dans le microbiote. La composition permet de rétablir la fonctionnalité métabolique par l'augmentation relative de *Christensenella* au sein de la population générale du microbiote soit au moins à un niveau représentant 0.01% du total des genres bactériens décelés dans le microbiote d'un individu donné (recueilli dans les selles, l'abondance étant mesurée de préférence par un test de séquençage Fish, ou de qPCR ou de méta analyse).

Cette augmentation permet de lutter contre les dysbioses pathologiques métaboliques causées par une déficience en *Christensenella* dans le microbiote intestinal.

L'augmentation de la quantité relative de *Christensenella* permet également de prévenir ou lutter contre des maladies métaboliques dont l'origine est une dysbiose pathologique métabolique caractérisée par une déficience relative en *Christensenella* dans le microbiote intestinal, et en particulier :
- le surpoids avec comorbidités
- l'obésité
- le diabète (et le pré-diabète)
- les maladies cardiaques et vasculaires
- l'athérosclérose
- les maladies dégénératives osseuses
- les maladies neurodégénératives, notamment maladie de Parkinson et maladie d'Alzheimer
- les cancers liés au métabolisme, notamment cancer de l'œsophage, du colon, de l'endomètre, du rein et du sein pour les femmes en post ménopause,
- les maladies auto-immunes,
- la stéatose, la stéatohépatite métabolique et NASH (stéatohépatite non alcoolique),
- les maladies inflammatoires chroniques de l'intestin.

La composition selon l'invention est particulièrement utile pour des personnes présentant une proportion relative de *Christensenella* inférieure à 0.01% du total des genres bactériens décelés dans le microbiote (recueilli dans les selles, l'abondance étant mesurée de préférence par un test de séquençage Fish, ou de qPCR ou de méta analyse), et pour des personnes présentant une proportion relative de *Christensenella minuta* dans le microbiote inférieure à 0.005% de l'abondance totale de la flore intestinale, c'est-à-dire du total des espèces bactériennes décelés dans le microbiote (recueilli dans les selles, l'abondance étant mesurée de préférence par un test de séquençage Fish, ou de qPCR ou de méta analyse).

Différentes méthodes peuvent être utilisées pour l'analyse du microbiote (le recueil se faisant généralement dans les selles) :
- la culture selon les méthodes classiques de culture bactérienne connues de l'homme du métier
- des méthodes biologiques à travers le marqueur 16SrRNA
- le séquençage soit par la méthode SANGER avec les Operational Taxonomic Units soit par spiroséquençage au niveau du 16SrRNA (méthode utilisée dans l'étude sur la composition)
- l'empreinte par électrophorèse, polymorphisme et ribosomal,
- ADN avec des micro aires,
- FISH (hybridation in situ e fluorescence) et qPCR (réaction en chaine par polymérase quantitative) avec amplification d'un groupe,
- Méta analyse avec la métagénomique (composition et fonction de tous les gènes), métaprotéomique (analyse des protéines), métabolomique (profil métabolique) et métatranscriptomique (à travers l'ARN).

Selon une variante, dans les cas extrêmes d'urgence médicale, il est possible, pour les personnes carencées en *Christensenella* d'implanter préalablement à l'administration de la composition selon l'invention des bactéries du genre *Christensenella* dans l'intestin, par l'utilisation d'un probiotique spécifique de cette bactérie en capsule ou par implantation directe par sonde dans le jéjunum. L'implantation devra représenter 75 à 100mg de bactérie *Christensenella.* Les bactéries implantées peuvent être des bactéries obtenues par culture de bactéries du genre *Christensenella* prélevées dans les selles de ladite personne traitée s'il en existe des traces ou des bactéries obtenues par culture de bactéries du genre *Christensenella* prélevées dans les selles de personnes maigres et en bonne santé.

L'utilisation de la composition selon l'invention dans des quantités adaptées permet, éventuellement avec implantation préalable, de porter la présence de *Christensenella* à une proportion relative minimale de 0.01 % par rapport à la totalité des taxons du microbiote recueilli dans les selles. La composition pourrait permettre également d'obtenir une quantité relative de *Christensenella minuta* (lorsque la méthode d'analyse permet de descendre jusqu'à l'espèce) d'au moins 0.0075% (par rapport à la totalité des taxons du microbiote recueilli dans les selles).

De façon surprenante la composition selon l'invention est également capable de réguler dans le microbiote les familles de bactéries connues pour leur présence relative importante dans le surpoids, l'obésité et les maladies métaboliques, choisies parmi les *clostridiales,* en particulier les *lachnospiraceae* et les *clostridiaceae.*

De manière étonnante également elle est aussi capable de réguler dans le microbiote les familles de bactéries connues pour leur capacité à augmenter la quantité d'énergie retirée des aliments, choisies parmi les bactéries qui donnent par fermentation le butyrate et le propionate mais aussi augmentent l'énergie par la digestion de fibres non digestes normalement, à savoir : *Roseburia, Faecalbacterium* (en particulier l'espèce *prausnitzii*), *Akkermansia* (en particulier l'espèce *municiphali*)*, Eubacterium* (en particulier l'espèce *rectale*)*, Méthanobrevibacter* (en particulier l'espèce *smithii*). Cette transformation supplémentaire d'énergie est l'une des causes la plus probable de l'épidémie de surpoids et de dysfonctionnement métabolique. Il est nécessaire de faire baisser ces « énergiseurs » mais sans atteindre une déficience car le microbiote est un grand consommateur de butyrate, propionate et acétate (par ordre d'importance) ; le genre *Akkermansia* et l'espèce *Akkermansia municiphali* (aussi héritables) sont particulièrement importants car ils sont les principaux producteurs de propionate qui est le garant d'un bon fonctionnement de la barrière intestinale en limitant le passage des LPS ; en effets ces derniers induits par les régimes trop gras (saturés en particulier) est le premier fautif du dysfonctionnement métabolique, de la stéatose hépatite et de la constitution de tissu adipeux. La régulation par l'invention de 2 gènes héritables et difficiles à faire évoluer est essentiel pour restaurer le bon fonctionnement du métabolisme et de servir de plus de marqueurs de la réussite de l'intervention du traitement par l'invention.

La composition selon l'invention peut se présenter sous toute forme adaptée à une administration par voie orale. Elle peut notamment se présenter sous forme de poudre ou granulés, de boissons prêtes à l'emploi, de barres ou d'extrudés, de capsules, de gélules, de comprimés dispersibles ou toute forme pharmaceutique adaptée, la composition étant additionnée d'excipients et charges classiques connues de l'homme du métier.

La dose journalière de composition selon l'invention (dose de mélange de principes actifs sans les excipients) est préférentiellement comprise entre 66 et 110 g, de préférence en deux prises de 33 à 55 g réparties une le matin au petit déjeuner ou à 11h en collation, et une en collation l'après-midi.

Avantageusement, dans le cadre d'un objectif de perte de poids, la biodisponibilité dans l'organisme des acides aminés, peptides et protéines présents dans la composition dans l'organisme est comprise entre 10 minutes et 5 heures, ce qui permet une action à la fois rapide et qui perdure dans le temps de façon à limiter la quantité de nourriture à prendre quotidiennement.

Par ailleurs, la présence de calcium de lait permet d'améliorer la palabilité du produit diététique selon l'invention en masquant notamment le goût amer de l'hydrolysat de lactosérum, de sorte qu'il participe à supprimer le risque que les personnes cessent de le consommer pour des raisons de goût et abandonnent leur traitement avant son terme.

La composition selon l'invention s'insère dans la médecine personnalisée et constitue une grande avancée en particulier dans la médecine pharmacométabonique.

La composition selon l'invention peut en effet être utilisée comme un traitement qui s'adapte au métagénome de la personne traitée.

Ainsi, avant le traitement par la composition selon l'invention, un test peut être réalisé pour détecter la présence ou non de *Christensenella* et sa proportion dans le microbiote. Ce test permet d'établir une probabilité de réussite totale ou partielle ou d'échec du traitement. Le test peut être reproduit à la fin du traitement pour analyser le niveau d'augmentation de la bactérie, puis à un an pour mesurer la pérennité du traitement et le danger de rechute avec la possibilité de refaire un traitement préventif. Le test peut porter également sur d'autres bactéries en particulier sur *Akkermansia.* L'avantage est de ne plus traiter à l'aveugle et de suivre à vie le patient dans sa maladie chronique.

L'invention permet ainsi de résoudre les problèmes des échecs thérapeutiques qui sont nombreux soit par l'abandon du traitement en cours et le plus souvent au début, soit par la reprise de poids après quelques mois ou années d'une perte de poids importante. Les échecs sont mis sur le compte du manque de motivation ou de lassitude de faire des efforts permanents mais le blocage peut être dû à la quasi absence ou absence de *Christensenella* dans le microbiote.

L'invention est à présent illustrée par un exemple non limitatif de composition diététique, se présentant sous forme d'une poudre de 55 g (principes actifs et excipients) conditionnée dans un sachet.

Cette composition est obtenue à partir des principes actifs suivants :
- 5g d'hydrolysat de lactosérum de poids moléculaire entre 200 et 3500 Daltons,
- 10 g d'isolat et/ou de concentrat de lactosérum de poids moléculaire entre 15000 et 20000 Daltons,
- 13 g de caséinate de calcium de poids moléculaire entre 20000 et 35000 Daltons,
- 1 mg de vitamine B6,
- 10 mg de zinc,
- 0,45 g de taurine,
- 40 µg de chrome,
- 10 mg de vitamine E,
- 100 µg de vitamine B9,
- 2,5 µg de vitamine D,
- 270 mg d'omégas 3 d'origine végétale,
- qsp 4,2g de leucine,
- qsp 0,8g de tryptophane,
- qsp 0,9g d'arginine,
- qsp 0,75g de calcium de lait,
- qsp 0,36 g de magnésium.

Par « qsp Xg » d'un élément de la composition, on entend la quantité totale de cet élément dans la composition : quantité apportée par les protides (caséinate de calcium, isolat de lactosérum, concentrat de lactosérum, hydrolysat de lactosérum) et complétée par un ajout de l'élément sous forme libre pour arriver jusqu'à Xg.

L'invention est à présent illustrée par une étude montrant l'effet de la composition sur le microbiote intestinal en particulier sur *Christensenella.*

L'étude a porté sur 54 sujets. Il s'agit d'une étude ancillaire d'une étude clinique internationale randomisée en aveugle avec un autre produit actif comme témoin. Deux études ancillaires ont été menées sur 54 sujets, l'une sur le microbiote et l'autre sur le tissu adipeux.

L'étude clinique portait sur 108 patients dont la caractéristique était d'être en surpoids ou obèses avec au moins 3 facteurs de comorbidités avérés ; les sujets étaient inclus lors d'une visite comprenant les critères morphologiques, un CTscan, une DEXA, une analyse biologique complète. Pour l'étude ancillaire concernant 54 sujets, un prélèvement du tissu adipeux sous cutanée et un recueil des selles étaient également réalisés. Les sujets recevaient ou la composition selon l'invention ou un témoin actif isocalorique correspondant à la composition selon l'invention mais dans laquelle le lactosérum était remplacé par une protéine de pois, connue pour son action sur le microbiote.

L'étude du microbiote comprenait un prélèvement à l'inclusion et l'autre au moment de la visite à la 12éme semaine à la fin du traitement du produit avec restriction calorique modérée. Les selles ont été congelées et à la fin de l'étude elles ont été analysées par piroséquence. Les analyses ont été menées par l'investigateur principal Institut du Cardiométabolisme et Nutrition de Paris.

Sur les 54 sujets, 27 ont été traités par la composition selon l'invention (composition de l'exemple) et 27 par le produit témoin.

Les selles ont été récoltées à l'inclusion et à la fin du traitement de 12 semaines avec les 2 produits et une restriction calorique de 600 kcal.

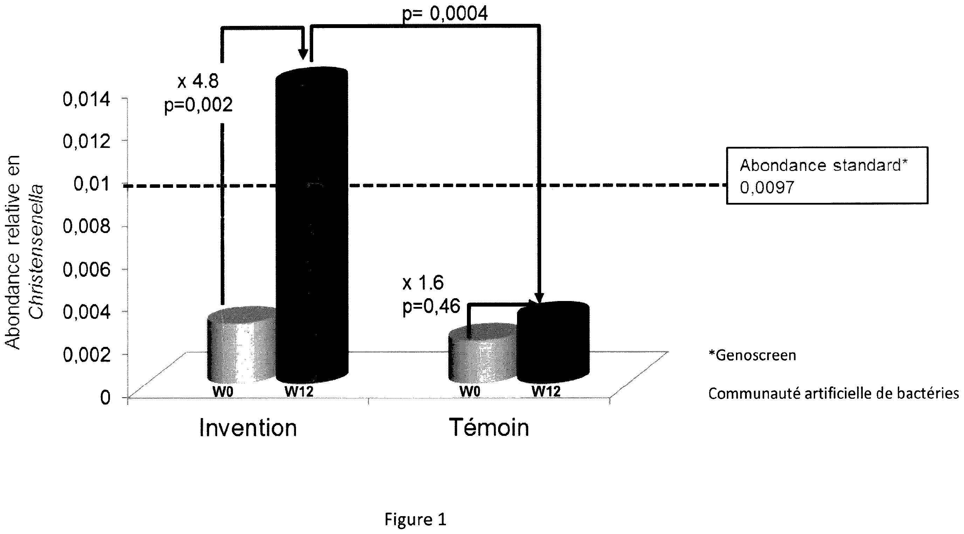

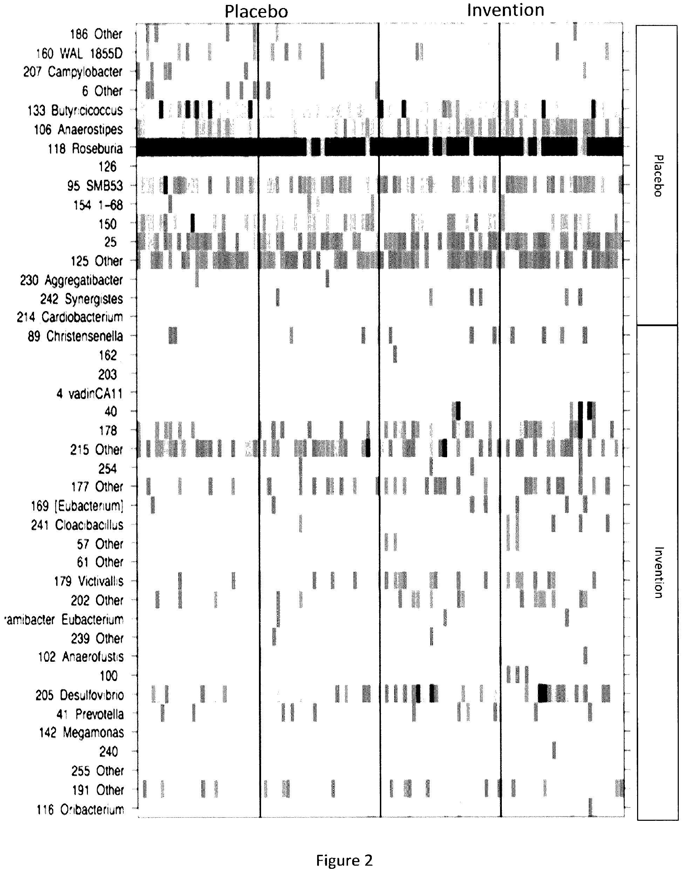

Les résultats pour l'étude du microbiote sont présentés dans le tableau 1 ci-après, et sur les figures 1 et 2.

Pour les résultats présentés sur la figure 2, il est à noter les éléments suivants :
- le test de Wilcoxon entre genres, a été réalisé sur la base d'une abondance normalisée,
- à la valeur p<0,05, 16 genres sont plus abondants avec le traitement placebo et 26 genres avec le traitement selon l'invention,
- seul un genre résiste aux multiples tests, *Christensenella.*

**Tableau 1 :**

| | STANDARD *Communauté artificielle de bactéries (1)* | | INVENTION | | | | | TEMOIN | | | | |
|---|---|---|---|---|---|---|---|---|---|---|---|---|
| | Présence relative | READS | PR W0 | PR W12 | READS W0 | READS W12 | Δ% | PR W0 | PR W12 | READS W0 | READS W12 | Δ% |
| POPULATION | | | | | 27 | | | | | | | |
| TOTAL TAXONS | 100 | 82394 | 100 | 100 | 75 875 | 74 220 | -1.5 | 100 | 100 | 73 838 | 72 365 | -1.2 |
| FIRMICUTES | 72.9 | 60019 | 67.2 | 68.2 | 51 260 | 50 633 | 12.0 | 69.3 | 63.5 | 51953 | 45 953 | -7.4 |
| BACTERIODETES | 20.7 | 17057 | 19.9 | 22.4 | 14 863 | 16 660 | 16 | 20.5 | 25.9 | 15 063 | 18 724 | 21.3 |
| PROTEOBACTERIA | 3.0 | 2470 | 7 | 4.8 | 5 430 | 3 538 | -20.6 | 5.8 | 6.8 | 4 281 | 4 962 | 16.5 |
| ACTINOBACTERIA | 5.0 | 4148 | 2.3 | 1.7 | 1 721 | 1 264 | -16.3 | 2.6 | 2.1 | 1896 | 1496 | -12.3 |
| FIRMICUTES/BACTERIODETES | | 3.5 | N | | 3.4 | 3.0 | | | | 3.4 | 2.5 | NS |
| | | | | | | | | | | | | |
| CONSORTIUM DU SURPOIDS (2) | 22 | 18 147 | 22.2 | 19.8 | 17 093 | 14 670 | -9 | 25.3 | 25.0 | 18 730 | 18 110 | 0.5 |
| CONSORTIUM ENERGISEURS (3) | 5.2 | 4 290 | 8.7 | 8.2 | 6 592 | 6073 | -6 | 10.5 | 12.0 | 7 801 | 8 670 | 13 |
| dont AKKERMANSIA | NC | NC | 0.0161 | 0.0337 | 12 | 25 | 118 | 0.0097 | 0.0186 | 7 | 13.5 | 57% |
| | | | | | | | | | | | | |
| CONSORTIUM DE CHRISTENSENELLACEAE (4) | 11.5 | 9 494 | 11.4 | 11.4 | 8 389 | 8 486 | 1.6 | 0.07 | 11.0 | 8 982 | 8 630 | -3.8 |
| **GENRE CHRISTENSENELLA** | **0.0097** | **8** | **0.0028** | **0.013** | **2** | **10** | **380** | **0.002** | **0.0027** | **1.5** | **2** | **60%** |
| | | | | | | | | | | | | |

| | | | | | | | | | | | | |
|---|---|---|---|---|---|---|---|---|---|---|---|---|
| *(1) il n'existe pas de modèle de microbiote idéal puisqu' il est individuel et dépend de la génétique et de l'acquis de chacun ; pour l'étude un standard des familles cultivées a été mis au point; ce standard a servi pour replacer les résultats dans un contexte* *(2) le consortium du surpoids comprend de nombreuses familles en particulier dans l'ordre des clostridiales mais 2 paraissent importantes pour leur nombre efforce d'impact : les lachnospiraceae et clostridiaceae* *(3) le consortium des* « *énergiseurs* » *sont les bactéries qui donnent par fermentation le butyrate et le propionate mais aussi augmentent l'énergie par la digestion de fibres non digestes normalement; il est composé de :Roseburia, Faecalbacterium, Akkermansia, Eubacterium, Méthanobrevibacter. Un équilibre dans ce groupe est particulièrement important.* *(4) le consortium de la christensenellaceae comprend les familles: Christensenellaceae, Méthanobacteriaceae, Dehalobacteriaceae, Peptococcaceae, Verrucomicrobiaceae, Clostridiales-non classifiées (dans la présente étude a été inclus clostridiales non classifiés et autres ce qui donne du poids à cette famille).* | | | | | | | | | | | | |

→ Sur le microbiote total il est à noter une pauvreté relative des 2 groupes Invention et Témoin par rapport au standard qui s'explique par la situation métabolique de ces 2 groupes ; le traitement n'améliore pas cette situation générale ce qui est logique puisque 12 semaines est une période de temps insuffisante pour les grandes phyla.
→ Sur la masse des grandes phyla traditionnelles firmicutes et bactériodetes et leur rapport, il existe une pauvreté relative mais surtout pour les firmicutes et ceci s'explique par la situation d'insulino résistance et inflammation très avancée ; la situation s'améliore légèrement en moyenne pour le groupe traité par la composition selon l'invention. Au niveau du rapport (actuellement controversé) la situation s'améliore mais d'avantage pour le groupe témoin à travers aussi d'une détérioration des firmicutes ce qui démontre le peu d'intérêt de ce rapport.
→ Dans les autres 2 grandes phyla, les *Protéobacteria* sont élevées et *Actinobacteria* faibles ; ces résultats traduisent parfaitement la situation métabolique de ces sujets ; il existe une évolution relative favorable pour le groupe de l'invention dans les 2 phyla.
→ Le consortium du surpoids évolue favorablement pour l'invention mais en sens contraire pour le témoin
→ Le consortium des énergiseurs qui est élevé dans les 2 groupes se redimensionne pour le groupe de l'invention contrairement au groupe témoin ; l'*Akkermansia* qui était particulièrement bas est redimensionné dans les 2 groupes mais avec une augmentation plus conséquente pour le groupe de l'invention.
→ Le consortium de la *Christensenellaceae* (Goodrich 2013) augmente dans les groupes traités selon l'invention, traduisant l'amélioration au niveau métabolique et sur la perte de poids.
→ Dans l'étude la famille *Christensenellaceae,* les genres non classifiés et n'appartenant pas à *Christensenella* sont primordiaux (>99%). Selon une publication de 2013 de Rajilic-Strojanovic, ces genres non classifiés seraient impliqués dans l'inflammation du « inflammatory bowel disease ». On constate ici une baisse en relatif pour un traitement selon l'invention de 1.26 à 1.12 et une augmentation pour le témoin de 0.83 à 0.89. Toutefois comme il devrait exister plusieurs genres dans cette partie non classifiée et qui pourraient évoluer de manière divergente il est impossible de tirer des conclusions ; il est prudent d'attendre de nouvelles découvertes dans ces genres non classifiés.
→ L'analyse du genre *Christensenella* est le noyau central de cette étude sur le microbiote.

*Christensenella* est une bactérie « héritable » et non pas acquise non sensible à la diète, dans cette catégorie elle semble être la plus importante du groupe. Ceci implique que pour les personnes qui ont un faible taux de *Christensenella* il est « vital » pour rétablir un métabolisme sain d'augmenter ce genre plutôt que l'ensemble du microbiote avec des prébiotiques ou des probiotiques qui peut provoquer des augmentations de classes indésirables comme les méthanogènes ou les hélicobacters. L'invention a une action ciblée sur les bactéries, utiles au métabolisme. La régulation d'*Akkermansia* arrive en deuxième priorité et ensuite celles sur le consortium du poids et des énergiseurs.

Par ailleurs, *Christensenella* est une bactérie marqueur d'un poids « normal » au point de vue métabolique et un marqueur de bonne santé donc d'un microbiote qui fonctionne régulièrement sans pathogénicité tant sur le plan génétique que mode de vie. De plus il a été démontré par Goodrich que l'implantation de *Christensenella* inversait la courbe de poids par une moindre prise de poids chez la souris ce qui se traduit chez l'homme par une perte de poids.

L'action de *Christensenella* est particulièrement essentielle pour une régulation métabolique afin de gommer les facteurs pathogènes qui se traduisent par des maladies métaboliques.

Le genre *Christensenella* est relativement sous représenté dans les 2 groupes (0.0028 et 0.002 contre 0.0097) par rapport au standard ce qui confirme la diminution de ce genre chez une population en souffrance métabolique. L'action des 2 produits fait augmenter *Christensenella* de 0.0028% à 0.014% pour le groupe de l'invention (380%) avec une significativité par rapport à l'initial (p=0.002), alors que le témoin passe de 0.002% à 0.0028% (+60% et non significatif). Le groupe traité selon l'invention passe en 12 semaines très nettement en dessus du standard (0.014% contre 0.0097%) et il est significatif par rapport au témoin (0.004).

Par ailleurs, il est à noter que de tous les genres que comportent le microbiote humain (1 500 connus à ce jour), le seul genre significatif dans l'étude sur l'invention qui sort de tous les tests statistiques est le *Christensenella* qui apparait comme un des genres le plus significatif des états pathologiques métaboliques du microbiote et des maladies métaboliques dérivées.

Par ailleurs, les résultats cliniques et de l'étude ancillaire sur le tissu adipeux sont présentés dans le tableau 2 ci-après :

**Tableau 2 :**

| | Composition selon l'invention | |
|---|---|---|
| | FAS TOTAL | FAS TOTAL |
| | Base Line | Δ% |
| ETUDE CLINIQUE | | |
| POPULATION | 108 | |
| MASSE GRASSE VISCERALE CTScan cm² | 192.3 | -10.4% |
| MASSE GRASSE TOTALE CTScan cm² | 489.7 | -8.9% |
| MASSE GRASSE DXA kg | 37.0 | -8.9% |
| MASSE MAIGRE DXA kg | 51.2 | -0.1% |
| TOUR DE TAILLE cm | 104.5 | -4.2% |
| POIDS kg | 92 | -3.9% |
| TAS mm | 131.7 | -2.7% |
| TAD mm | 81.6 | -4.0% |
| GLYCÉMIE mmol | 5.5 | -2.8% |
| INSULINEMIE mUl/l | 12.6 | -19.0% |
| HOMA IR | 3.1 | -20.5% |
| HOMA B | 124 | -9.3% |
| HOMA S | 59.9 | +40.6% |
| CHOLESTEROL TOTAL mmol | 5.2 | -7.2% |
| HDL mmol | 1.2 | +0.2% |
| LDL mmol | 3.2 | -7.6% |
| TRIGLYCERIDES mmol | 1.6 | -9.6% |
| TNF pg/ml | 1.9 | -10.2% |

| ETUDE ANCILAIRE TISSU ADIPEUX | | |
|---|---|---|
| GLYCEROL pg/ml | 16 | -20.1% |
| IL6 | 3707 | -13.3% |
| 0² CONSOMMATION ENERGIE µM | 0.002 | +63% |
| ADIPONECTINE pg/ml | 11426 | +13.3% |
| | | |

On constate que la composition utilisée selon l'invention, a une action certaine sur la fonction métabolique de l'adipocyte qui se traduit par l'augmentation de l'adiponectine dans le tissu adipeux qui reflète les deux dysfonctions de base qui sont à l'origine des nombreuses maladies métaboliques et dégénératives :
- l'inflammation avec une baisse de l'IL6 dans le tissu adipeux et le TNFα dans le plasma ceci est expliqué par l'activation de p105 sur les adipocytes qui régule NPκB le modulateur des cytokines inflammatoires
- l'insulino résistance qui s'améliore par la baisse de l'insulinémie, la baisse de HOMA IR mais surtout une amélioration très sensible de la sensibilité à l'insuline par HOMA S
- deux autres fonctions sur le tissu adipeux sont à noter la régulation de la lipolyse des adipocytes des personnes en surpoids (glycérol) et l'augmentation de la fabrication d'énergie (consommation O²)
- l'amélioration des facteurs de comorbidités : tension artérielle, glycémie, insulinémie, cholestérol, LDL et triglycérides, est à la fois une conséquence de l'amélioration métabolique mais aussi de l'action directe des composés actifs de l'invention en particulier des acides aminés
- enfin par ailleurs aucun effet secondaire sérieux n'a été relevé lors du traitement hormis quelques embarras digestifs passagers.

Selon l'invention, il est ainsi possible d'agir sur une dysbiose pathologique de la flore intestinale à travers l'augmentation relative du genre *Christensenella,* et par conséquent agir également sur les maladies métaboliques marquées par cette pathologie. Les actions sur d'autres familles, genres ou espèces viennent renforcer l'action de *Christensenella.*

## Revendications

1. Composition comprenant un mélange de principes actifs constitué par au moins :
- un hydrolysat de lactosérum de poids moléculaire compris entre 200 et 10000 Daltons,
- un isolat et/ou un concentrat de lactosérum de poids moléculaire compris entre 15 000 et 20 000 Daltons, et
- du caséinate de calcium,
pour son utilisation comme médicament ou produit de nutrition médicale chez l'homme pour le traitement d'une dysbiose pathologique du microbiote intestinal **caractérisée par** une déficience en *Christensenella* dans le microbiote intestinal.

2. Composition pour son utilisation selon l'une des précédentes revendications, chez des personnes dont le genre *Christensenella* représente moins de 0,01% du total des genres bactériens décelés dans le microbiote inestinal.

3. Composition pour son utilisation selon la revendication 2, **caractérisée en ce que** l'espèce bactérienne *Christensenella* est du genre bactérien *Christensenella minuta.*

4. Composition pour son utilisation selon la revendication 3, chez des personnes présentant une proportion relative de *Christensenella minuta* dans le microbiote inférieure à 0.005% de l'abondance totale de la flore intestinale.

5. Composition pour son utilisation selon l'une des revendications 3 ou 4, de façon à réguler dans le microbiote les bactéries connues pour leur capacité à augmenter la quantité d'énergie retirée des aliments, choisies parmi les genres *Roseburia, Faecalbacterium, Akkermansia, Eubacterium, Méthanobrevibacter.*

6. Composition pour son utilisation selon l'une des précédentes revendications pour des personnes en carence de *Christensenella* et chez qui ces bactéries du genre *Christensenella* ont été préalablement implantées dans l'intestin par l'utilisation d'un probiotique spécifique de cette bactérie en capsule ou par implantation directe par sonde.

7. Composition pour son utilisation selon la revendication 6, **caractérisée en ce que** les bactéries implantées chez la personne traitée sont des bactéries obtenues par culture de bactéries du genre *Christensenella* prélevées dans l'intestin de ladite personne traitée.

8. Composition pour son utilisation selon la revendication 6, **caractérisée en ce que** les bactéries implantées chez la personne traitée sont des bactéries obtenues par culture de bactéries du genre *Christensenella* dont la souche a été prélevée dans l'intestin de personnes maigres et en bonne santé.

9. Composition pour son utilisation selon l'une des précédentes revendications, **caractérisée en ce que** le caséinate de calcium a un poids moléculaire compris entre 20 000 et 35 000 Daltons.

10. Composition pour son utilisation selon l'une des précédentes revendications, **caractérisée en ce que** l'hydrolysat de lactosérum a un poids moléculaire compris entre 200 et 3500 Daltons.

11. Composition pour son utilisation selon l'une des précédentes revendications, **caractérisée en ce que** le ratio en poids entre le caséinate de calcium et le mélange constitué par l'hydrolysat et l'isolat et/ou le concentrat de lactosérum est compris entre 0,8 et 1,2.

12. Composition pour son utilisation selon l'une des précédentes revendications, **caractérisée en ce que** le mélange de principes actifs comprend également un mélange d'acides aminés.

13. Composition pour son utilisation selon la revendication 12, **caractérisée** en que le mélange d'acides aminés est constitué par au moins un des acides aminés choisis parmi le tryptophane, la glutamine, la leucine, l'arginine et la taurine.

14. Composition pour son utilisation selon la revendication 13, **caractérisée** en que le tryptophane représente entre 6 et 9% en poids des acides aminés neutres présents dans la composition.

15. Composition pour son utilisation selon l'une des revendications 13 ou 14, **caractérisée en ce que** le rapport du poids d'arginine sur le poids de taurine est compris entre 1,5 et 2.

16. Composition pour son utilisation selon l'une des précédentes revendications, **caractérisée en ce que** le mélange de principes actifs comprend également au moins un des éléments choisis parmi le calcium de lait, le magnésium, la vitamine B6, la vitamine B9, la vitamine E, la vitamine D, le zinc et le chrome.

17. Composition pour son utilisation selon l'une des précédentes revendications, **caractérisée en ce que** le mélange de principes actifs comprend également des acides gras essentiels.

18. Composition pour son utilisation selon la revendication 17, **caractérisée en ce que** les acides gras essentiels sont des omégas 3.

19. Composition pour son utilisation selon l'une des précédentes revendications, **caractérisée en ce que** l'hydrolysat de lactosérum représente entre 8 et 12%, l'isolat et/ou le concentrat de lactosérum entre 15 et 20%, et le caséinate de calcium entre 20 et 25%, les pourcentages étant donnés en poids de matière sèche de la totalité des principes actifs présents de la composition.

20. Composition pour son utilisation selon l'une des précédentes revendications, **caractérisée en ce qu'**elle est constituée par au moins :
- 1,5 à 3% de tryptophane,
- 12 à 20 % d'acides aminés branchés,
- 6 à 10 % d'acides aminés aromatiques,
- 0,8 à 1,5% de taurine,
- 1,6 à 3% d'arginine,
- 1,2 à 3% de calcium de lait,
- 0,5 à 1% de magnésium,
- 0,4 à 1% d'omégas 3,
- 1 à 2 mg de vitamine B6 pour 50g de composition sans excipients,
- 5 à 15 mg de zinc pour 50g de composition sans excipients,
- 1 à 3 µg de vitamine D pour 50g de composition sans excipients,
- 75 à 150 µg de chrome pour 50g de composition sans excipients,
- 100 µg de vitamine B9 pour 50g de composition sans excipients,
- 10 mg de vitamine E pour 50g de composition sans excipients,
les pourcentages étant donnés en poids de matière sèche de la totalité des principes actifs présents de la composition.

21. Composition pour son utilisation selon l'une des précédentes revendications, **caractérisée en ce qu'**elle se présente sous forme de poudre ou granulés, de boisson prête à l'emploi, de barres alimentaires ou extrudés, de capsules ou gélules ou de comprimés dispersibles.

## Patentansprüche

1. Zusammensetzung, umfassend eine Mischung von Wirkstoffen, bestehend aus mindestens:
- einem Molkenhydrolysat mit einem Molekulargewicht zwischen 200 und 10.000 Dalton,
- einem Molkenisolat und/oder -konzentrat mit einem Molekulargewicht zwischen 15.000 und 20.000 Dalton und
- Calciumcaseinat,
zur Verwendung als Medikament oder medizinisches Ernährungsprodukt beim Menschen zur Behandlung der pathologischen Dysbiose der intestinalen Mikrobiota, die durch einen Mangel an *Christensenella* in der intestinalen Mikrobiotagekennzeichnet ist.

2. Zusammensetzung zur Verwendung nach einem der vorhergehenden Ansprüche bei Personen, bei denen die Gattung *Christensenella* weniger als 0,01 % der Gesamtheit der in der Darmflora nachgewiesenen Bakteriengattungen ausmacht.

3. Zusammensetzung zur Verwendung nach Anspruch 2, **dadurch gekennzeichnet, dass** die Bakterienart *Christensenella* zur Bakteriengattung *Christensenella minuta gehört.*

4. Zusammensetzung zur Verwendung nach Anspruch 3 bei Personen, die einen relativen Anteil von *Christensenella minuta* in der Mikrobiota von weniger als 0,005 % der Gesamthäufigkeit der Darmflora aufweisen.

5. Zusammensetzung zur Verwendung nach einem der Ansprüche 3 oder 4, um in der Mikrobiotadie Bakterien zu regulieren, die für ihre Fähigkeit bekannt sind, die aus der Nahrung entnommene Energiemenge zu erhöhen, ausgewählt aus den Gattungen *Roseburia, Faecalbacterium, Akkermansia, Eubacterium, Methanobrevibacter.*

6. Zusammensetzung zur Verwendung nach einem der vorhergehenden Ansprüche für Personen mit einem *Christensenella-Mangel* und bei denen diese Bakterien der Gattung *Christensenella* zuvordurch Verwendung eines spezifischen Probiotikums für dieses Bakterium in Kapseln oder durch direkte Einsetzung per Sonde in den Darm eingesetzt wurden.

7. Zusammensetzung zur Verwendung nach Anspruch 6, **dadurch gekennzeichnet, dass** die in die behandelte Person eingesetzten Bakterien Bakterien sind, die durch Kultivieren von Bakterien der Gattung *Christensenella* erhalten wurden, die aus dem Darm der behandelten Person entnommen wurden.

8. Zusammensetzung zur Verwendung nach Anspruch 6, **dadurch gekennzeichnet, dass** die in die behandelte Person eingesetzten Bakterien Bakterien sind, die durch Kultivieren von Bakterien der Gattung *Christensenella* erhalten wurden, deren Stamm aus dem Darm dünner und gesunder Menschen entnommen wurde.

9. Zusammensetzung zur Verwendung nach einem der vorhergehenden Ansprüche, **dadurch gekennzeichnet, dass** das Calciumcaseinat ein Molekulargewicht zwischen 20.000 und 35.000 Dalton aufweist.

10. Zusammensetzung zur Verwendung nach einem der vorhergehenden Ansprüche, **dadurch gekennzeichnet, dass** das Molkenhydrolysat ein Molekulargewicht zwischen 200 und 3500 Dalton aufweist.

11. Zusammensetzung zur Verwendung nach einem der vorhergehenden Ansprüche, **dadurch gekennzeichnet, dass** das Gewichtsverhältnis zwischen dem Calciumcaseinat und der Mischung, die aus dem Molkenhydrolysat und dem -isolat und/oder dem Molkenkonzentrat besteht, zwischen 0,8 und 1,2 liegt.

12. Zusammensetzung zur Verwendung nach einem der vorhergehenden Ansprüche, **dadurch gekennzeichnet, dass** die Mischung von Wirkstoffen auch eine Mischung von Aminosäuren umfasst.

13. Zusammensetzung zur Verwendung nach Anspruch 12, **dadurch gekennzeichnet, dass** die Mischung von Aminosäuren aus mindestens einer der Aminosäuren besteht, die aus Tryptophan, Glutamin, Leucin, Arginin und Taurin ausgewählt sind.

14. Zusammensetzung zur Verwendung nach Anspruch 13, **dadurch gekennzeichnet, dass** das Tryptophan zwischen 6 und 9 Gew.-% der in der Zusammensetzung vorhandenen neutralen Aminosäuren ausmacht.

15. Zusammensetzung zur Verwendung nach einem der Ansprüche 13 oder 14, **dadurch gekennzeichnet, dass** das Gewichtsverhältnis von Arginin zu Taurin zwischen 1,5 und 2 liegt.

16. Zusammensetzung zur Verwendung nach einem der vorhergehenden Ansprüche, **dadurch gekennzeichnet, dass** die Mischung von Wirkstoffen auch mindestens eines der Elemente umfasst, die aus Milchcalcium, Magnesium, Vitamin B6, Vitamin B9, Vitamin E, Vitamin D, Zink und Chrom ausgewählt sind.

17. Zusammensetzung zur Verwendung nach einem der vorhergehenden Ansprüche, **dadurch gekennzeichnet, dass** die Mischung von Wirkstoffen auch essentielle Fettsäuren umfasst.

18. Zusammensetzung zur Verwendung nach Anspruch 17, **dadurch gekennzeichnet, dass** die essentiellen Fettsäuren Omega-3-Fettsäuren sind.

19. Zusammensetzung zur Verwendung nach einem der vorhergehenden Ansprüche, **dadurch gekennzeichnet, dass** das Molkenhydrolysat zwischen 8 und 12 %, das Molkenisolat und/oder das -konzentrat zwischen 15 und 20 % und das Calciumcaseinat zwischen 20 und 25 % ausmacht, wobei die Prozentsätze als Gewicht der Trockenmasse aller in der Zusammensetzung vorhandenen Wirkstoffe angegeben sind.

20. Zusammensetzung zur Verwendung nach einem der vorhergehenden Ansprüche, **dadurch gekennzeichnet, dass** sie besteht aus mindestens:
- 1,5 bis 3 % Tryptophan,
- 12 bis 20 % verzweigten Aminosäuren,
- 6 bis 10 % aromatischen Aminosäuren,
- 0,8 bis 1,5 % Taurin,
- 1,6 bis 3 % Arginin,
- 1,2 bis 3 % Milchcalcium,
- 0,5 bis 1 % Magnesium,
- 0,4 bis 1 % Omega 3,
- 1 bis 2 mg Vitamin B6 pro 50 g Zusammensetzung ohne Hilfsstoffe,
- 5 bis 15 mg Zink pro 50 g Zusammensetzung ohne Hilfsstoffe,
- 1 bis 3 µg Vitamin D pro 50 g Zusammensetzung ohne Hilfsstoffe,
- 75 bis 150 µg Chrom für 50 g Zusammensetzung ohne Hilfsstoffe,
- 100 µg Vitamin B9 für 50 g Zusammensetzung ohne Hilfsstoffe,
- 10 mg Vitamin E für 50 g Zusammensetzung ohne Hilfsstoffe,
wobei die Prozentsätze als Gewicht der Trockenmasse aller in der Zusammensetzung vorhandenen Wirkstoffe angegeben sind.

21. Zusammensetzung zur Verwendung nach einem der vorhergehenden Ansprüche, **dadurch gekennzeichnet, dass** sie in Form von Pulver oder Granulat, eines gebrauchsfertigen Getränks, von Extrudaten oder Lebensmittelriegeln, von Kapseln oder Gelatinekapseln oder von dispergierbaren Tabletten vorliegt.

## Claims

1. Composition comprising a mixture of active principles consisting of at least:
- a whey hydrolyzate having a molecular weight of between 200 and 10,000 Daltons,
- a whey isolate and/or concentrate having a molecular weight between 15,000 and 20,000 Daltons, and
- calcium caseinate,
for use as a drug or medical nutrition product in humans for the treatment of pathological dysbiosis of the intestinal microbiota, **characterized by** a deficiency of *Christensenella* in the intestinal microbiota.

2. Composition for use according to any of the preceding claims, in persons in whom the genus *Christensenella* represents less than 0.01 % of the total of the bacterial genera detected in the intestinal microbiota.

3. Composition for use according to claim 2, **characterized in that** the bacterial species *Christensenella* is of the bacterial genus *Christensenella minuta.*

4. Composition for use according to claim 3, in persons having a relative proportion of *Christensenella minuta* in the microbiota of less than 0.005% of the total abundance of the intestinal flora.

5. Composition for use according to either claim 3 or claim 4, so as to regulate in the microbiota bacteria known for their ability to increase the amount of energy withdrawn from food, selected from the genera *Roseburia, Faecalbacterium, Akkermansia, Eubacterium, Methanobrevibacter.*

6. Composition for use according to any of the preceding claims for persons lacking *Christensenella* and in whom these bacteria of the genus *Christensenella* have been previously implanted in the intestines by the use of a probiotic specific to this bacterium in capsule form or by direct implantation by probe.

7. Composition for use according to claim 6, **characterized in that** the bacteria implanted in the treated person are bacteria obtained by culturing bacteria of the genus *Christensenella* taken from the intestines of said treated person.

8. Composition for use according to claim 6, **characterized in that** the bacteria implanted in the treated person are bacteria obtained by culturing bacteria of the genus *Christensenella,* the strain of which has been taken from the intestines of lean and healthy persons.

9. Composition for use according to any of the preceding claims, **characterized in that** the calcium caseinate has a molecular weight of between 20,000 and 35,000 Daltons.

10. Composition for use according to any of the preceding claims, **characterized in that** the whey hydrolyzate has a molecular weight of between 200 and 3500 Daltons.

11. Composition for use according to any of the preceding claims, **characterized in that** the ratio by weight between the calcium caseinate and the mixture consisting of the whey hydrolyzate and the whey isolate and/or concentrate is between 0.8 and 1.2.

12. Composition for use according to any of the preceding claims, **characterized in that** the mixture of active principles also comprises a mixture of amino acids.

13. Composition for use according to claim 12, **characterized in that** the mixture of amino acids consists of at least one of the amino acids selected from tryptophan, glutamine, leucine, arginine and taurine.

14. Composition for use according to claim 13, **characterized in that** the tryptophan represents between 6 and 9% by weight of the neutral amino acids present in the composition.

15. Composition for use according to either claim 13 or claim 14, **characterized in that** the ratio of the weight of arginine to the weight of taurine is between 1.5 and 2.

16. Composition for use according to any of the preceding claims, **characterized in that** the mixture of active principles also comprises at least one of the elements selected from milk calcium, magnesium, vitamin B6, vitamin B9, vitamin E, vitamin D, zinc and chromium.

17. Composition for use according to any of the preceding claims, **characterized in that** the mixture of active principles also comprises essential fatty acids.

18. Composition for use according to claim 17, **characterized in that** the essential fatty acids are omega 3.

19. Composition for use according to any of the preceding claims, **characterized in that** the whey hydrolyzate represents between 8 and 12%, the whey isolate and/or concentrate represents between 15 and 20%, and the calcium caseinate represents between 20 and 25%, the percentages being given by weight of dry matter of all the active principles present in the composition.

20. Composition for use according to any of the preceding claims, **characterized in that** it consists of at least:
- 1.5 to 3% tryptophan,
- 12 to 20% branched amino acids,
- 6 to 10% aromatic amino acids,
- 0.8 to 1.5% taurine,
- 1.6 to 3% arginine,
- 1.2 to 3% milk calcium,
- 0.5 to 1% magnesium,
- 0.4 to 1% omega 3,
- 1 to 2 mg of vitamin B6 per 50 g of composition without excipients,
- 5 to 15 mg of zinc per 50 g of composition without excipients,
- 1 to 3 µg of vitamin D per 50 g of composition without excipients,
- 75 to 150 µg of chromium per 50 g of composition without excipients,
- 100 µg of vitamin B9 per 50 g of composition without excipients,
- 10 mg of vitamin E per 50 g of composition without excipients,
the percentages being given by weight of dry matter of all the active principles present in the composition.

21. Composition for use according to any of the preceding claims, **characterized in that** it is provided in the form of a powder or granules, a ready-to-use beverage, food bars or extrudates, capsules or dispersible tablets.
